# EUROPEAN PATENT SPECIFICATION

(11) **EP 2 593 459 B1**
(45) Date of publication and mention of the grant of the patent: **24.09.2014**
(21) Application number: 11743210.4
(22) Date of filing: 12.07.2011
(51) Int. Cl.: C07D 487/04, C07D 239/557, A61K 31/4985, A61P 3/10

(54) **PROCESS FOR THE PREPARATION OF SITAGLIPTIN OROTATE**
VERFAHREN ZUR HERSTELLUNG VON ORGANISCHEN SALZEN
PROCÉDÉ DE PRÉPARATION DE SELS ORGANIQUES

(30) Priority: 13.07.2010 EP 10169397
(43) Date of publication of application: 22.05.2013
(73) Proprietor: Chemo Iberica, S.A., 08025 Barcelona (ES)
(72) Inventor: VENTIMIGLIA, Gianpiero, I-72021 Francavilla Fontana (IT); MAGRONE, Domenico, 37138 Verona (IT); BRAVIN, Fabio Massimo, I-20126 Milano (IT)
(74) Representative: Palladino, Saverio Massimo
(86) International application number: PCT/EP2011/061842
(87) International publication number: WO 2012/007455

(56) References cited:
- WO-A1-2005/072530
- WO-A2-2009/085990
- WO-A2-2010/000469
- WO-A2-2010/012781
- WO-A2-2010/117738

## Description

### Field of the invention

The present invention relates to a novel process for the preparation of sitagliptin orotate in amorphous form.

### Background of the invention

Sitagliptin, 7-[(3*R*)-3-amino-1-oxo-4-(2,4,5-trifluorophenyl)butyl]-5,6,7,8-tetrahydro-3-(trifluoromethyl)-1,2,4-triazolo[4,3-*a*]pyrazine, is the compound of formula (I):

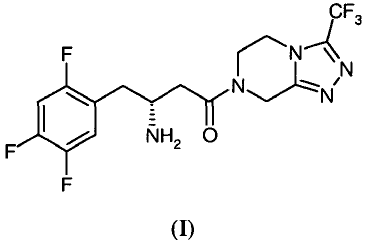

Sitagliptin, disclosed in US Pat. No. 6,699,871 B2 and in international application WO 03/004498 A1, belongs to a class of beta-amino tetrahydrotriazolo-[4,3-*a*]pyrazines, which are potent second generation inhibitors of dipeptidyl-peptidase IV (DP-IV) useful for the treatment of Type-2 diabetes. The preparation of the compound is also described in the paper "Highly Efficient Asymmetric Synthesis of Sitagliptin", Hansen et al.: Journal of the American Chemical Society (2009), 131(25), 8798-8804; once obtained, the compound can be purified according to methods standard in the field, obtaining sitagliptin of purity suitable for pharmaceutical applications.

Sitagliptin is marketed as monophosphate salt and monohydrate adduct under the trade name Januvia^{®} (Trademark of Merck & Co.).

The discovery of novel pharmaceutically acceptable salts of active pharmaceutical ingredients (APIs) in their amorphous forms provides a new opportunity to improve the performance characteristics of a pharmaceutical product. It enlarges the repertoire of materials that a formulation scientist has available for designing a pharmaceutical dosage form, or a drug with a targeted release profile, or other desired physico-chemical characteristics, such as density, melting point, colour, stability, dissolution rate, milling facility, granulation, compacting, flowability and suitable rate of dissolution in aqueous fluid. Further, amorphous forms of APIs can provide new commercial opportunities for a pharmaceutical company. Finally, use of eco-compatible chemicals and processes, suitable for large scale preparations, is desirable.

Several polymorphic forms are reported for the salts of sitagliptin in patent literature; see, e.g., WO 2005/072530 A1, WO 2009/085990 A2, WO 2010/000469 A2 and WO 2010/012781 A2.

Among them, the phosphoric acid salt of sitagliptin in amorphous form has already been disclosed in patent application WO 2006/033848 A1. However, regarding large scale preparations, disposal of phosphate containing wastes could be a problem for environmental purposes.

Moreover, amorphous form of salt of sitagliptin with orotic acid has been disclosed in patent application US 2010/0249140 A1. According to those skilled in the art, the process described in said application is affected by several drawbacks:
a) the amorphous salt is obtained by a precipitation mixing between sitagliptin free base and orotic acid in acetonitrile, thus giving rise to a sticky gel very difficult to stir, handle and filter;
b) a stoichiometric ratio between sitagliptin free base and orotic acid cannot be guaranteed under these conditions;
c) due to the poor filterability of the obtained product, it's not reasonable in a large scale preparation to distil the solvent under normal or reduced pressure to obtain the dry solid, both for quality or safety purposes;
d) a residual amount of acetonitrile, which is no compatible with regulatory purposes, is still present in the final product.

On the basis of these considerations, a novel process for the preparation of amorphous salt of sitagliptin with orotic acid is desirable.

An object of the present invention is to provide a novel process for the preparation of sitagliptin with orotic acid, herein below referred to as sitagliptin orotate.

Finally, the potential for polymorphism may also give rise to distinct spectroscopic properties that may be detectable by X-Ray Powder Diffraction (XRPD) and Differential Scanning Calorimetry (DSC) analysis.

### Summary of the invention

The present invention relates to a novel process for the preparation of amorphous salt of sitagliptin with orotic acid, characterized by the following improvements:
a) a higher eco-compatibility process;
b) a stoichiometric ratio between sitagliptin and orotic acid is guaranteed (excess sitagliptin free base is removed by means of washing with an organic solvent);
c) a higher suitability for large-scale preparations.

In one embodiment, the invention provides a process for preparing sitagliptin orotate comprising the steps of:
a) suspending sitagliptin free base in water;
b) adding orotic acid in a ratio 0.9:1 to 0.95:1 mole/mole with respect to sitagliptin;
c) heating the solution up to a temperature comprised in the range 30 °C to 40 °C in order to promote the reaction of orotic acid with sitagliptin free base;
d) cooling down or allowing to cool the solution to a temperature comprised in the range of 15 °C to 25 °C;
e) extracting residual sitagliptin free base by means of an organic solvent immiscible with water;
f) separating the aqueous phase and filtering it;
g) lyophilizing the aqueous solution.

### Brief description of the drawings and instrumental skills

Figure 1 provides a DSC thermogram of amorphous sitagliptin orotate;
Figure 2 provides a XRPD pattern of amorphous sitagliptin orotate.

### Detailed description of the invention

All terms as used in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The present inventors have now found that sitagliptin may be isolated as a salt with orotic acid by means of a new suitable, economic, efficient and eco-compatible process, particularly suited for large-scale preparation. Orotic acid has the IUPAC name 1,2,3,6-tetrahydro-2,6-dioxo-4-pyrimidinecarboxylic acid and formula (II):

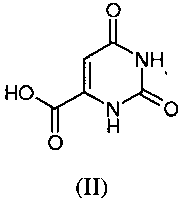

In the conditions of the process of the invention, orotic acid protonates the amino group of sitagliptin, giving rise to a salt formed by ionic attraction between the sitagliptin-derived cation and the orotate anion in stoichiometric ratio. The resulting salt can be represented by formula (III) below, in which the dotted line indicates the zone of attraction between the two ions:

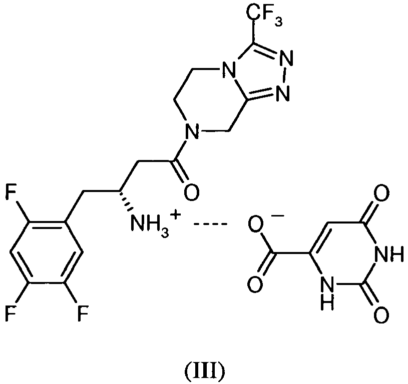

In one embodiment, the invention provides a process for the preparation of sitagliptin orotate comprising the steps of:
a) suspending sitagliptin free base in water at a suitable temperature, comprised in the range of about 10 °C to about 30 °C; preferably, the temperature is comprised in the range of about 15 °C to about 25 °C;
b) adding orotic acid. Orotic acid can be used either anhydrous or, preferably, as monohydrate adduct; orotic acid is in a ratio of 0.9:1 to 0.95:1 mole/mole with respect to sitagliptin;
c) heating the solution obtained in step b) up to a temperature comprised in the range of 30 °C to 40 °C in order to promote the reaction between orotic acid and sitagliptin free base;
d) cooling down the solution with suitable means, or allowing the solution to freely cool down, to a temperature comprised in the range of 15 °C to 25 °C;
e) extracting residual sitagliptin free base by means of an organic solvent immiscible with water;
f) separating the aqueous phase by means of methods well known to those skilled in the art and filtering it;
g) lyophilizing the aqueous solution thus obtained by means of methods well known to those skilled in the art.

In step a), the concentration of sitagliptin free base in water is preferably comprised in the range of 6% up to 20% weight/weight; operating with a concentration lower than 6% the yield of the process is too low, while operating with a concentration in excess of 20% gives rise to problems in product handling in step g) of the process.

The organic solvent immiscible with water for the extraction of step e) is selected from the following classes: aromatics (preferably toluene), chlorinated (preferably dichloromethane), esters, linear or branched aliphatic esters (preferably ethyl acetate, isopropyl acetate, n-butyl acetate, isobutyl acetate), ethers, preferably dialkyl ethers, linear or branched, preferably acyclic (diethylether, methyl *tert*-butyl ether (MTBE), diisopropylether), alcohols (preferably 1-butanol, 2-butanol, *tert*-butanol), ketones, linear or branched aliphatic ketones (preferably methyl ethyl ketone, methyl isobutyl ketone). Preferably, the organic solvent is used in a ratio of 1:4 to 1:6 volume/volume with respect to water.

The aqueous phase obtained through separation in step f) is filtered before submitting it to the subsequent lyophilizing step in order to carefully get rid of particles that could damage the lyophilization apparatus, as known to the experts in the field. Filtering can be carried out by any known methods, preferably through a celite pad or a 0.45 µm filter.

Sitagliptin orotate obtained by the process of the present invention shows a residual water content comprised in a range of about 3.5% to about 5.5% by weight, as determined by means of Karl Fischer titration.

The invention is further illustrated by means of one example.

### Example 1

Sitagliptin free base, having HPLC purity of 99.9% and with an enantiomeric excess of the 3*R*-isomer higher than 99.9%, is prepared according to the Hansen *et al.* paper cited before. 1.6 g (3.9 mmol) of the compound are suspended in water (20 ml) at 20 °C and orotic acid monohydrate (0.64 g, equivalent to 3.7 mmol of anhydrous orotic acid, Sigma-Aldrich) is added. The solution is warmed up to 35-37 °C, kept at this temperature for ten minutes, and then allowed to cool down spontaneously to 24-25 °C. The system is then extracted twice with dichloromethane (5 ml), and the resulting aqueous phase is separated, filtered through a celite pad and lyophilized, thus affording amorphous sitagliptin orotate (2.1 g), having a water content of 4.7% by weight as determined by Karl Fischer titration.

On the thus obtained product, XRPD analysis and DSC thermal analysis are carried out.

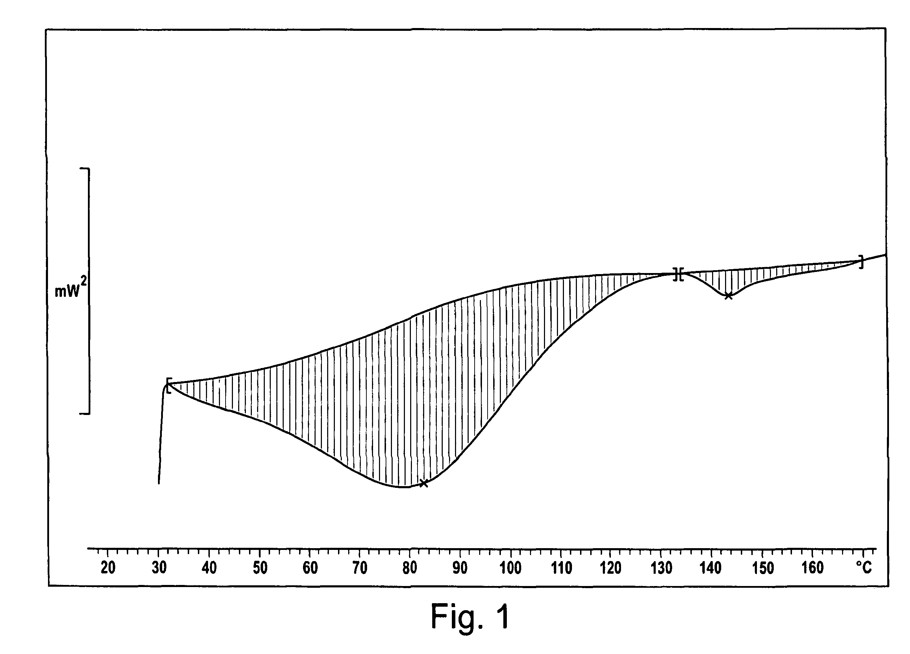

XRPD analysis is performed on a APD 2000 Ital Structures diffractometer at room temperature, using a CuKα tube (40 kV, 30 mA, λ, = 1.5406 Å) as the X-ray source. Data collection is made in 2θ step scan mode, at a scan speed of 0.04°/s in the range of 3° to 40° in 2θ, using as sample approximately 100 mg of product accurately ground and placed on an aluminium sampler. The results of this test are reported in Figure 1, which shows the typical pattern of an amorphous material.

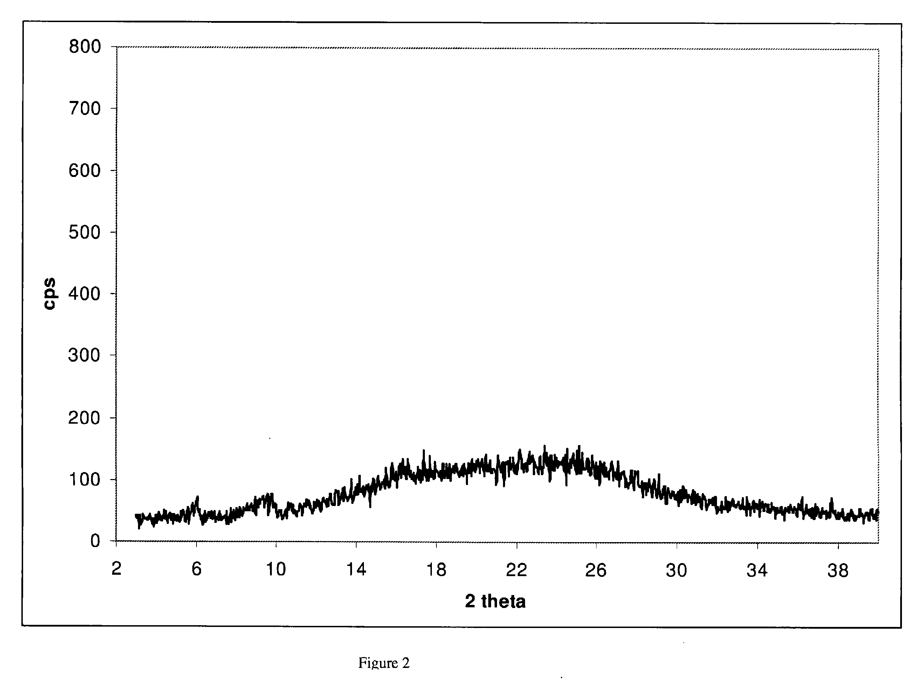

DSC thermal analysis is performed on a Mettler Toledo STAR^{e} 822 differential scanning calorimeter. A sample of approximately 5 mg is placed in an aluminium crimped pan and heated from 30 to 170 °C in a dry nitrogen atmosphere at a heating rate of 10 °C/minute. The results of this test are reported in Figure 2. In the figure, the upper line of the graph is the "base line" of the test, namely, it represents the heat exchanged by an empty sample holder (equal to the one holding the sample under test), while the lower line represents the heat exchanged by the sample holder; the difference between these two lines represents the contribution to heat exchange of the sample, and the integral of the areas defined between the two lines is a measure of the heat exchanged by the sample. The DSC thermogram evidences a broad endothermic feature with a maximum peak at about 83 °C due to the loss of water; additionally, it's noteworthy to observe a thermal phenomenon at about 144 °C, due to a glass transition or a similar phenomenon.

## Claims

1. Process for the preparation of sitagliptin orotate in amorphous form, comprising the steps of:
a) suspending sitagliptin free base in water;
b) adding orotic acid in a ratio 0.9:1 to 0.95:1 mole/mole with respect to sitagliptin;
c) heating the solution up to a temperature comprised in the range 30 °C to 40 °C in order to promote the reaction of orotic acid with sitagliptin free base;
d) cooling down or allowing to cool the solution to a temperature comprised in the range 15 °C to 25 °C;
e) extracting residual sitagliptin free base by means of an organic solvent immiscible with water;
f) separating the aqueous phase and filtering it;
g) lyophilizing the aqueous solution.

2. Process according to claim 1, wherein in step a) the concentration of sitagliptin free base in water is comprised in the range of 6% up to 20% weight/weight.

3. Process according to any one of the preceding claims, wherein the organic solvent used in step e) is selected from the following classes: aromatic compounds, chlorinated compounds, esters, ethers, alcohols and ketones.

4. Process according to claim 3, wherein said solvent is chosen among linear or branched aliphatic esters, linear or branched dialkyl ethers and linear or branched aliphatic ketones.

5. Process according to claim 4, wherein said solvent is chosen among toluene, dichloromethane, ethyl acetate, isopropyl acetate, n-butyl acetate, isobutyl acetate, diethylether, methyl *tert*-butyl ether (MTBE), diisopropylether, 1-butanol, 2-butanol, tert-butanol, methyl ethyl ketone and methyl isobutyl ketone.

6. Process according to any one of the preceding claims, wherein the organic solvent is used in a ratio 1:4 to 1:6 volume/volume with respect to water.

7. Process according to any one of the preceding claims, wherein filtering of step f) is carried out by means of a celite pad or a 0.45 µm filter.

## Patentansprüche

1. Verfahren zum Herstellen von Sitagliptinorotat in amorpher Form, welches die nachfolgenden Schritte umfasst:
a) Suspendieren von freier Sitagliptinbase in Wasser,
b) Zugabe von Orotsäure in einem Verhältnis von 0,9:1 bis 0,95:1 mol/mol bezogen auf Sitagliptin,
c) Erhitzen der Lösung auf eine Temperatur zwischen 30°C und 40°C, um die Reaktion von Orotsäure mit freier Sitagliptinbase zu fördern,
d) Herunterkühlen oder Erlauben des Abkühlens der Lösung auf eine Temperatur in einem Bereich von 15°C bis 25°C,
e) Extrahieren von restlicher freier Sitagliptinbase mittels eines organischen Lösungsmittels, welches mit Wasser nicht mischbar ist,
f) Abtrennen der wässrigen Phase und Filtrieren derselben,
g) Lyophilisieren der wässrigen Lösung.

2. Verfahren nach Anspruch 1, wobei in dem Schritt a) die Konzentration von freier Sitagliptinbase in Wasser in einem Bereich zwischen 6% bis 20% Gewicht/Gewicht liegt.

3. Verfahren nach einem der vorhergehenden Ansprüche, wobei das organische Lösungsmittel, welches in dem Schritt e) eingesetzt wird, aus den nachfolgenden Klassen ausgewählt wird: aromatische Verbindungen, chlorierte Verbindungen, Estern, Ethern, Alkoholen und Ketonen.

4. Verfahren nach Anspruch 3, wobei das Lösungsmittel aus linearen oder verzweigten aliphatischen Estern, linearen oder verzweigten Dialkylethern und linearen oder verzweigten aliphatischen Ketonen ausgewählt wird.

5. Verfahren nach Anspruch 4, wobei das Lösungsmittel ausgewählt wird aus Toluol, Dichlormethan, Etyhlacetat, Isopropylacetat, n-Butylacetat, Isobutylacetat, Diethylether, Methyl-tert-butylether (MTBE), Diisopropylether, 1-Butanol, 2-Butanol, tert-Butanol, Methylethylketon und Methylisobutylketon.

6. Verfahren nach einem der vorhergehenden Ansprüche, wobei das organische Lösungsmittel in einem Verhältnis von 1:4 bis 1:6 Volumen/Volumen bezogen auf Wasser eingesetzt wird.

7. Verfahren nach einem der vorhergehenden Ansprüche, wobei das Filtrieren in dem Schritt f) mittels eines Celite-Blocks oder eines 0,45 µm Filters durchgeführt wird.

## Revendications

1. Procédé de préparation d'orotate de sitagliptine sous forme amorphe, comprenant les étapes de :
a) mise en suspension d'une base libre de sitagliptine dans de l'eau ;
b) addition d'acide orotique dans un rapport de 0,9:1 à 0,95:1 en mole par rapport à la sitagliptine ;
c) chauffage de la solution jusqu'à une température comprise dans la plage de 30°C à 40°C afin de favoriser la réaction de l'acide orotique avec la base libre de sitagliptine ;
d) refroidissement ou permission de refroidissement de la solution à une température comprise dans la plage de 15°C à 25°C ;
e) extraction de la base libre de sitagliptine résiduelle à l'aide d'un solvant organique non miscible avec l'eau ;
f) séparation de la phase aqueuse et filtration de celle-ci ;
g) lyophilisation de la solution aqueuse.

2. Procédé selon la revendication 1, dans lequel, dans l'étape a), la concentration de la base libre de sitagliptine dans l'eau est comprise dans la plage de 6 % jusqu'à 20 % en poids.

3. Procédé selon l'une quelconque des revendications précédentes, dans lequel le solvant organique utilisé dans l'étape e) est choisi parmi les catégories suivantes : les composés aromatiques, les composés chlorés, les esters, les éthers, les alcools et les cétones.

4. Procédé selon la revendication 3, dans lequel ledit solvant est choisi parmi les esters aliphatiques linéaires ou ramifiés, les dialkyléthers linéaires ou ramifiés et les cétones aliphatiques linéaires ou ramifiées.

5. Procédé selon la revendication 4, dans lequel ledit solvant est choisi parmi le toluène, le dichorométhane, l'acétate d'éthyle, l'acétate d'isopropyle, l'acétate de n-butyle, l'acétate d'isobutyle, le diéthyléther, le méthyl-tert-butyléther (MTBE), le diisopropyléther, le 1-butanol, le 2-butanol, le tert-butanol, la méthyléthylcétone et la méthylisobutylcétone.

6. Procédé selon l'une quelconque des revendications précédentes, dans lequel le solvant organique est utilisé dans un rapport de 1:4 à 1:6 en volume par rapport à l'eau.

7. Procédé selon l'une quelconque des revendications précédentes, dans lequel la filtration de l'étape f) est réalisée à l'aide d'un tampon de célite ou d'un filtre de 0,45 µm.
